# EUROPEAN PATENT APPLICATION

(11) **EP 4 137 140 A1**
(43) Date of publication of application: **22.02.2023**
(21) Application number: 21788272.9
(22) Date of filing: 12.04.2021
(51) Int. Cl.: A61K 35/28, A61K 9/00, A61P 17/00, C12N 5/0775

(54) **COMPOSITION FOR PREVENTING OR TREATING DIABETIC SKIN DISEASE, COMPRISING EXOSOME DERIVED FROM THROMBIN-TREATED STEM CELL**

(30) Priority: 14.04.2020 KR 20200045483
(71) Applicant: Samsung Life Public Welfare Foundation, Seoul 04348 (KR)
(72) Inventor: CHANG, Yun Sil, Songpa-gu, Seoul 05649 (KR); PARK, Won Soon, Myeongdal-ro Seoul 06713 (KR); SUNG, Dong Kyung, Nowon-gu, Seoul 01618 (KR)
(74) Representative: Ostertag & Partner Patentanwälte mbB
(86) International application number: PCT/KR2021/004597
(87) International publication number: WO 2021/210872

(57) **Abstract**

The present invention relates to: a pharmaceutical composition which is for preventing or treating diabetic skin disease, and comprises, as an active ingredient, an exosome derived from a thrombin-treated stem cell; a pharmaceutical formulation containing said composition; and a method for producing same. In the present invention, an exosome isolated from a thrombin-treated stem cell was found to have an excellent effect on the recovery of diabetic animal models from diabetic dermatopathy. In addition, since exosome-based therapeutics are cell-free formulations and thus do not include DNA, such therapeutics have a low risk of cancer and are free of cell surface antigens. Accordingly, exosome-based therapeutics do not have the issue of transplant rejection and are thus safe. Moreover, being separation materials rather than cells, exosome-based therapeutics can be developed as off-the-shelf drugs, and thus reduce manufacturing costs. Accordingly, the exosome derived from a thrombin-treated stem cell according to the present invention can be developed as a therapeutic agent for various intractable chronic skin diseases that can be caused by diabetes, and furthermore, is expected to be useful for application to fields related to skin regeneration.

## Description

### [Technical Field]

The present invention relates to a pharmaceutical composition for preventing or treating a diabetic skin disease, which includes an exosome isolated from thrombin-treated stem cells as an active ingredient, a pharmaceutical formulation containing the composition, and a method of preparing the same.

### [Background Art]

Diabetes is a type of metabolic disease in which insulin secretion is insufficient or normal function is not achieved, characterized by high blood sugar in which the concentration of glucose in the blood increases, and causes various symptoms and signs due to high blood sugar and excretion of glucose in urine. It is known that type I diabetes also known as "juvenile diabetes" is caused by an absolute or relative lack of insulin production due to the destruction of beta cells of the pancreas by an autoimmune mechanism, whereas type 2 diabetes is induced by insulin resistance and caused by various factors such as obesity, a decrease in insulin receptors, a decrease in insulin secretion, and genetic factors.

According to the 2016 announcement of the Korean Diabetes Association, as of 2014, the prevalence of diabetes among Korean adults aged 30 or higher was recorded as 13.7%, an all-time high, and the risk of diabetic comorbidities was also serious. The prevalence of obesity and abdominal obesity in diabetic patients reached 48.6% and 58.9%, respectively, and high blood pressure and hypercholesterolemia accompanied at 54.7% and 31.6%, respectively. As such, diabetes can cause serious complications such as cardiovascular diseases, retinopathy, renal failure, peripheral neuropathy and skin diseases as well as its own risks.

A diabetic skin disease, which is one of the diseases caused by diabetes, may be caused by maintaining a high glucose level in blood for a long time, and specifically includes diabetic ulcers, acanthosis nigricans, necrobiosis lipoidica diabeticorum, diabetic dermatopathy, eruptive xanthomatosis, soft fibroma, pruritus, granuloma annulare, and bacterial and fungal infections. Particularly, in the case of dermatosis, recovery is not good due to a high blood sugar concentration, so it can become a chronic disease. Therefore, it is necessary to develop effective formulations that can prevent and treat various skin diseases caused by diabetes.

Meanwhile, stem cells are known as cells involved in tissue regeneration, treatment and immune responses along with their pluripotency, and therefore there are efforts to develop a therapeutic agent for various diseases and symptoms by isolating and culturing mesenchymal stem cells from cord blood and bone marrow using the above characteristics.

However, treatment using stem cells themselves has had the following limitations and side effects. First, basically, in the case of a cell therapeutic agent, the possibility of tumorigenicity due to DNA transfer cannot be excluded, and second, due to the large size of stem cells themselves, it can cause vascular obstruction or myocardial infarction, third, when transplanting using allogeneic cells such as cord blood (allograft), there is a problem of rejection due to a cell surface antigen, and fourth, in general, a cell therapeutic agent has limitations in that the manufacturing process is difficult and the production cost is high due to restrictions on storage and transportation. As such, due to inherent limitations of stem cells, efficacy improvement methods using genetic manipulation have been developed as methods for reducing side effects and improving therapeutic effects, but there is no clear alternative to date.

Exosomes are small vesicles (diameter: approximately 30 to 100 nm) having a membrane structure secreted from various cells, and in research using an electron microscope, it has been observed that they originate from specific intracellular compartments called multivesicular bodies (MVBs), rather than directly detached from the plasma membrane, and are released and secreted out of the cells. That is, when the MVB and the plasma membrane are fused, the vesicles are released to the extracellular environment, called exosomes. Although it is not clear by what molecular mechanism these exosomes are produced, various types of immune cells including red blood cells, as well as B-lymphocytes, T-lymphocytes, dendritic cells, platelets, and macrophages, tumor cells and stem cells are known to produce and secrete exosomes while they are alive.

Particularly, exosomes derived from stem cells contain not only a receptor and a protein, but also a nuclear component, and are known to play a role in intracellular communication. In addition, the stem cell-derived exosomes contain relatively less animal serum compared to stem cells, so the risk of zoonosis caused by animal serum infection can also be excluded. Considering these characteristics of exosomes, cell therapy using exosomes is expected to be a new paradigm that can overcome the limitations of conventional stem cell therapies.

In this regard, the therapeutic effect on diabetic skin ulcers using pluripotent stem cells has been disclosed (KR10-2018-0104196), but the use of thrombin-treated stem cell-derived exosomes for the treatment of a diabetic skin disease is not yet known.

### [Disclosure]

### [Technical Problem]

As a result of research efforts by applying stem cell-derived exosomes to a diabetic skin disease, the inventors confirmed an excellent therapeutic effect by the exosomes when skin lesions were induced in a diabetic animal model as an example of diabetic skin disease. Based on this, the present invention was completed.

Therefore, the present invention is directed to providing a pharmaceutical composition for preventing or treating a diabetic skin disease, which includes thrombin-treated stem cell-derived exosomes as an active ingredient.

In addition, the present invention is directed to providing a pharmaceutical formulation for treating a diabetic skin disease, which includes the pharmaceutical composition.

In addition, the present invention is directed to providing a quasi-drug formulation for improving a diabetic skin disease, which includes thrombin-treated stem cell-derived exosomes.

In addition, the present invention is directed to providing a method of preparing the pharmaceutical composition.

However, technical problems to be solved in the present invention are not limited to the above-described problems, and other problems which are not described herein will be fully understood by those of ordinary skill in the art from the following descriptions.

### [Technical Solution]

To achieve the purpose of the present invention, the present invention provides a pharmaceutical composition for preventing or treating a diabetic skin disease, which includes thrombin-treated stem cell-derived exosomes as an active ingredient.

In one embodiment of the present invention, the stem cells may be selected from the group consisting of mesenchymal stem cells, human tissue-derived mesenchymal stromal cells, human tissue-derived mesenchymal stem cells, pluripotent stem cells, and amniotic epithelial cells.

In another embodiment of the present invention, the mesenchymal stem cells may be derived from the umbilical cord, cord blood, bone marrow, fat, muscle, nerves, skin, the amniotic membrane or placenta.

In still another embodiment of the present invention, the diabetic skin disease may be selected from the group consisting of diabetic ulcers, eruptive xanthomatosis, cutaneous infection, diabetic dermopathy, skin fibroma, acanthosis nigricans, pruritus, scleredema diabeticorum, and granuloma annulare.

In yet another embodiment of the present invention, the pharmaceutical composition may further include an auxiliary component selected from the group consisting of a culture medium, a cytokine, a growth factor, and a gene.

In addition, the present invention provides a pharmaceutical formulation for treating a diabetic skin disease.

In one embodiment of the present invention, the pharmaceutical formulation may be an injectable type, an infusion type, a spray type, a liquid type, or a patch type.

In addition, the present invention provides a quasi-drug formulation for improving a diabetic skin disease, which includes thrombin-treated stem cell-derived exosomes as an active ingredient.

In one embodiment of the present invention, the quasi-drug formulation may be an external formulation for skin selected from the group consisting of a liquid, an ointment, a cream, a spray, a patch, a gel, and an aerosol.

In addition, the present invention provides a method of preparing the pharmaceutical composition, which includes the following steps:
(a) treating thrombin after stem cell culture;
(b) isolating exosomes from the cell culture of (a); and
(c) preparing a composition containing the exosomes isolated in (b) as an active ingredient.

In one embodiment of the present invention, the thrombin of (a) may be contained at 1 to 1000 unit/ml in the medium.

In another embodiment of the present invention, the exosomes of (b) may be isolated by centrifugation at 3,000 to 100,000 g for 10 minutes to 5 hours.

In addition, the present invention provides a method of preventing or treating a diabetic skin disease, which includes administering a pharmaceutical composition including thrombin-treated stem cell-derived exosomes as an active ingredient to an individual.

In addition, the present invention provides a use of the pharmaceutical composition for preventing or treating a diabetic skin disease.

### [Advantageous Effects]

In the present invention, it was confirmed according to a specific embodiment of the present invention that exosomes isolated from thrombin-treated stem cells have an excellent diabetic dermatopathy recovery effect in diabetic animal models. In addition, since exosome-based therapeutic agents are cell-free formulations and thus do not include DNA, such therapeutic agents have a low risk of cancer and are free of cell surface antigens. Accordingly, exosome-based therapeutic agents do not have the issue of transplant rejection and are thus safe. Moreover, as a separation material, rather than cells, exosome-based therapeutic agents can be developed as off-the-shelf products, and thus reduce production costs. Accordingly, the exosome derived from a thrombin-treated stem cell according to the present invention can be developed as a therapeutic agent for various intractable chronic skin diseases that can be caused by diabetes, and furthermore, is expected to be effectively useful for application to fields related to skin regeneration.

### [Description of Drawings]

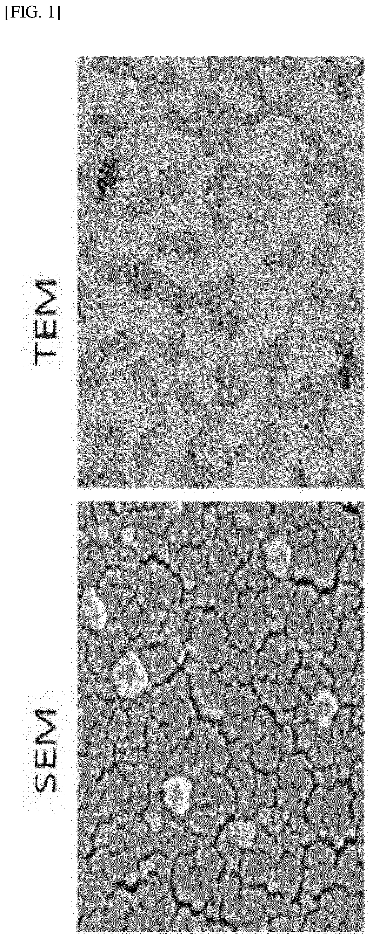

FIG. 1 is a set of images of exosomes isolated from thrombin-treated stem cells obtained by a scanning electron microscope (SEM) and a transmission electron microscope (TEM).

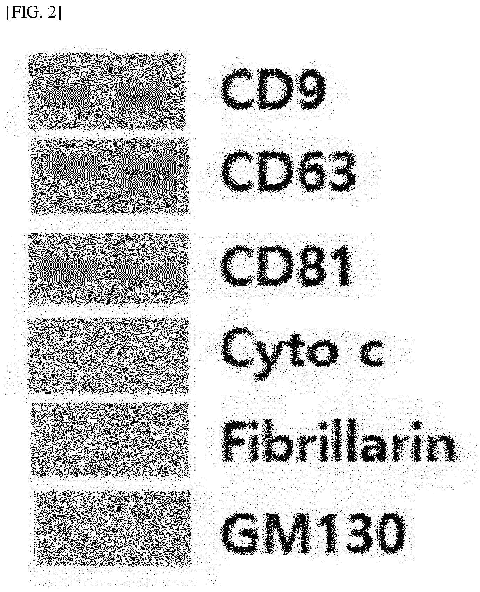

FIG. 2 is a result of measuring levels of exosome markers CD9, CD63 and CD81 through western blotting to confirm that a product isolated from thrombin-treated stem cells is an exosome.

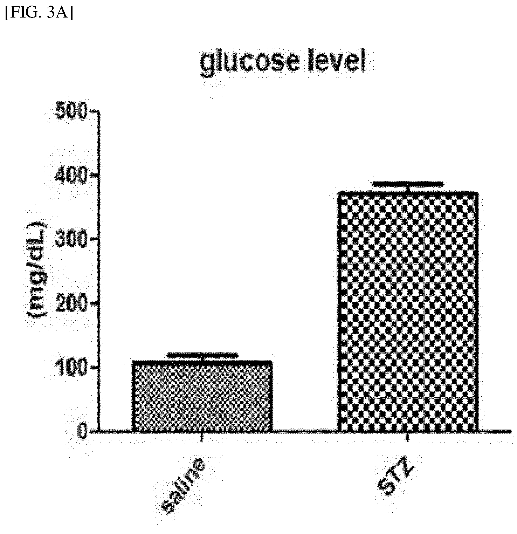

FIG. 3A shows the result of manufacturing a diabetes-induced animal model for verifying a therapeutic effect of thrombin-treated stem cell-derived exosomes on diabetic dermatopathy, and average blood glucose levels of a normal group (Saline) prepared by intraperitoneally administering saline into a rat and a group (STZ) in which diabetes is induced by intraperitoneal administration of streptozotocin.

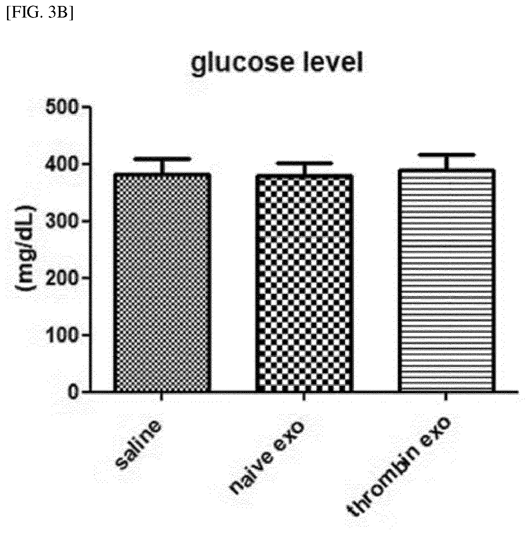

FIG. 3B is a result showing that there is no difference in blood glucose between experimental groups in which diabetic dermatopathy is induced and treated with saline (Saline), untreated stem cell-derived exosomes (naive exo), and exosomes isolated from thrombin-treated mesenchymal stem cells (thrombin exo).

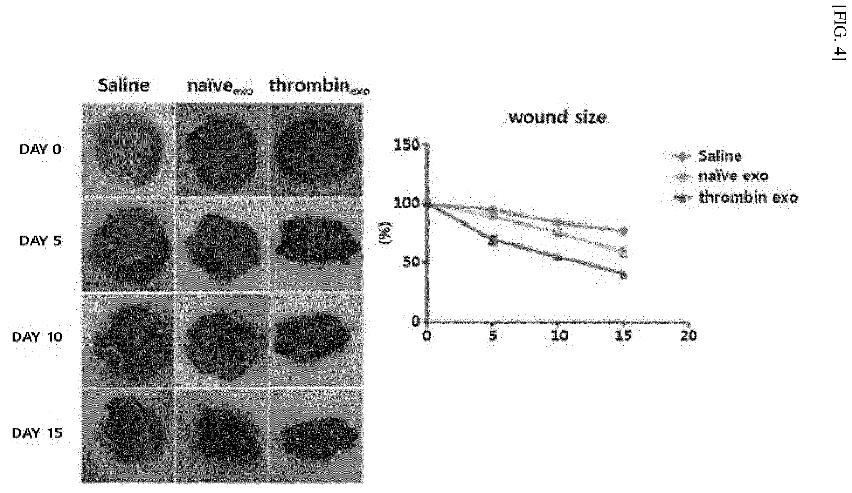

FIG. 4 is a result of comparing wound healing effects every 5 days when saline (Saline), untreated stem cell-derived exosomes (naive exo), and exosomes isolated from thrombin-treated mesenchymal stem cells (thrombin exo) are directly applied to wounds, respectively, immediately after making wounds in diabetic-induced rat models to cause dermatopathy.

### [Modes of the Invention]

As a result of research efforts by applying stem cell-derived exosomes derived from stem cells to a diabetic skin disease, the inventors confirmed an excellent therapeutic effect by the exosomes when skin lesions were induced in a diabetic animal model as an example of diabetic skin disease. Based on this, the present invention was completed.

Therefore, the present invention provides a pharmaceutical composition for preventing or treating a diabetic skin disease, which includes thrombin-treated stem cell-derived exosomes as an active ingredient.

The term "thrombin" is a protease involved in blood coagulation, and serves to catalyze a reaction of changing soluble fibrinogen in blood to insoluble fibrin through hydrolysis. In the present invention, it is applied to stem cells to effectively enhance the therapeutic effect of exosomes isolated from the stem cells on a diabetic skin disease.

The "stem cell" used herein refers to an "undifferentiated cell" which has differentiation ability but has not yet differentiated, and a cell that has self-replication ability and the ability to differentiate into two or more different types of cells. The stem cells of the present invention may be autologous or allogeneic stem cells, and may be derived from any type of animal, such as a human and a non-human mammal.

In the present invention, the stem cells are embryonic stem cells or adult stem cells, and preferably adult stem cells. The adult stem cells may be mesenchymal stem cells, human tissue-derived mesenchymal stromal cells, human tissue-derived mesenchymal stem cells, or pluripotent stem cells, and preferably mesenchymal stem cells, but the present invention is not limited thereto.

The mesenchymal stem cells may be mesenchymal stem cells derived from the umbilical cord, cord blood, bone marrow, fat, muscle, nerves, skin, the amniotic membrane or placenta, and preferably cord blood-derived mesenchymal stem cells, but the present invention is not limited thereto.

The term "cord blood" refers to blood collected form the umbilical vein connecting the placenta and a fetus. Cord blood, a naturally occurring by-product during delivery, is much easier to collect than general mesenchymal tissue such as bone marrow, which requires multiple surgeries, and compared to bone marrow transplantation, it is easy to find a donor because the cord blood storage industry has been activated and the infrastructure has already been established. In addition, since cord blood-derived cells are cells that do not express the histocompatibility antigen HLA-DR (class II), which is the most important cause of rejection in tissue or organ transplantation, it does not induce or minimizes the immune response, such as rejection, which has been a problem during conventional transplant surgery, so not only autologous cord blood but also heterologous cord blood can be used.

The term "exosome" used herein refers to a small vesicle (diameter: approximately 30 to 100 nm) having a membrane structure secreted from various cells, and means a vesicle released to an extracellular environment by fusion between the MVB and a plasma membrane. The exosomes include those that are naturally secreted, or those that are artificially secreted.

The term "prevention" used herein refers to all actions of inhibiting a diabetic skin disease or delaying the onset thereof by administration of the pharmaceutical composition according to the present invention.

The term "treatment" used herein refers to all actions involved in alleviating or beneficially changing symptoms of a diabetic skin disease by administration of the pharmaceutical composition according to the present invention.

The term "diabetic skin disease" used herein includes various diseases associated with the skin due to an increase in blood sugar caused by insulin resistance and abnormal insulin secretion, and specifically may be selected from the group consisting of diabetic ulcers, eruptive xanthomatosis, cutaneous infection, diabetic dermopathy, skin fibroma, acanthosis nigricans, pruritus, scleredema diabeticorum, and granuloma annulare, but the present invention is not limited thereto.

The pharmaceutical composition of the present invention may further include an auxiliary component selected from the group consisting of a culture medium, a cytokine, a growth factor, and a gene.

The pharmaceutical composition of the present invention may be used alone or in combination with methods using surgery, radiation therapy, hormone therapy, chemotherapy, and a biological response modifier for the treatment of a diabetic skin disease.

The pharmaceutical composition of the present invention may include thrombin-treated stem cell-derived exosomes as an active ingredient, and further include a pharmaceutically acceptable carrier. The pharmaceutically acceptable carrier is generally used in formulation, and includes saline, distilled water, Ringer's solution, buffered saline, cyclodextrin, a dextrose solution, a maltodextrin solution, glycerol, ethanol, liposomes, etc., but the present invention is not limited thereto. If needed, the pharmaceutically composition may further include other conventional additives including an antioxidant, a buffer, etc. In addition, by additionally adding a diluent, a dispersant, a surfactant, a binder or a lubricant, the pharmaceutical composition may be formulated as an injectable form such as an aqueous solution, an emulsion or a suspension, a pill, a capsule, a granule or a tablet. Suitable pharmaceutically acceptable carriers and their formulations may be formulated according to each ingredient using a method disclosed in the Remington's Pharmaceutical Science.

The pharmaceutical composition of the present invention may be administered orally or parenterally (e.g., intravenously, subcutaneously, intraperitoneally or locally) depending on a desired method, and a dose of the pharmaceutical composition may vary depending on the condition and body weight of a patient, the severity of a disease, a drug type, an administration route and time, and may be suitably selected by one of ordinary skill in the art.

The pharmaceutical composition is administered at a pharmaceutically effective amount. The term "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field. The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without a side effect, and such a dose may be easily determined by one of ordinary skill in the art.

Specifically, the effective amount of the pharmaceutical composition of the present invention may vary according to a patient's age, sex, condition, body weight, absorption rate of an active ingredient in the body, an inactivation rate, an excretion rate, a disease type or a concomitant drug, and generally, 0.001 to 150 mg, and preferably 0.01 to 100 mg per kg of body weight may be administered daily or every other day, or one to three times a day. However, the effective amount may be increased or decreased depending on the route of administration, the severity of obesity, sex, a body weight or age, and thus it does not limit the scope of the present invention in any way.

In addition, the present invention provides a pharmaceutical formulation for treating a diabetic skin disease, which includes the pharmaceutical composition.

The pharmaceutical formulation may be an injectable type, an infusion type, a spray type, a liquid type or a patch type, but the present invention is not limited thereto. A suitable form for the treatment of a corresponding diabetic skin disease may be selected by those of ordinary skill in the art.

According to a specific embodiment of the present invention, it was confirmed that thrombin-treated stem cell-derived exosomes specifically have an excellent therapeutic effect on a diabetic skin disease.

In one embodiment of the present invention, cord blood-derived mesenchymal stem cells were treated with thrombin and cultured, and then exosomes were isolated through centrifugation. Subsequently, the isolated exosomes were observed under a scanning electron microscope and a transmission electron microscope, and it was confirmed that exosomes are well isolated by measuring the expression levels of exosome-specific markers CD9, CD63 and CD81 (see Example 1).

In another embodiment of the present invention, before evaluating the therapeutic effect of the exosome on a diabetic skin disease, it was confirmed that diabetes was effectively induced compared to the normal group by measuring the blood glucose of a diabetes-induced rat model. Accordingly, in a diabetic rat model having the same level of blood sugar without a blood sugar difference, a wound was made on the dorsal skin to induce dermatopathy followed by treatment with each of saline, untreated stem cell-derived exosomes, or thrombin-treated stem cell-derived exosomes and evaluation of the degree of dermatosis recovery every 5 days. As a result, it was confirmed that there is a significant therapeutic effect when the thrombin-treated stem cell-derived exosome according to the present invention is treated (see Example 2).

Therefore, as another aspect of the present invention, the present invention provides a quasi-drug formulation for improving a diabetic skin disease, which includes thrombin-treated stem cell-derived exosomes.

The quasi-drug formulation in the present invention may be an external formulation for skin selected from the group consisting of a liquid, an ointment, a cream, a spray, a patch, a gel, and an aerosol, and specifically, can be used as a disinfectant, a shower foam, a mouth wash, a wet tissue, a laundry soap, a hand wash, a humidifier filter, a mask, an ointment or a filter filler composition, but the present invention is not limited thereto. The quasi-drug formulation may be suitably selected by those of ordinary skill in the art and used to improve a diabetic skin disease.

In still another aspect of the present invention, the present invention provides a method of preparing the pharmaceutical composition, which includes: (a) treating thrombin after stem cell culture; (b) isolating exosomes from the cell culture of (a); and (c) preparing a composition containing the exosomes isolated in (b) as an active ingredient.

In the present invention, the thrombin treatment concentration is not particularly limited as long as it is a concentration suitable for enhancing the efficacy of stem cells/exosomes, but thrombin is preferably included at 1 to 1000 unit/ml in the medium.

In the present invention, the method of isolating exosomes is not limited, and for example, exosomes may be isolated from a culture through centrifugation, ultracentrifugation, filtration using a filter, gel filtration chromatography, free-flow electrophoresis, capillary electrophoresis, or isolation using a polymer alone, or in combination thereof, and preferably, centrifugation/ultracentrifugation. Here, centrifugation/ultracentrifugation is preferably performed at 4 °C and 3,000 to 100,000 g for 10 minutes to 5 hours.

In the present invention, the medium used in cell culture refers to a mixture for the growth and proliferation of stem cells in vitro, which includes elements essential for cell growth and proliferation, such as sugar, amino acids, various types of nutrients, serum, growth factors, and minerals. The medium that can be used herein includes commercially produced media such as Dulbecco's Modified Eagle's Medium (DMEM), Minimal Essential Medium (MEM), Basal Medium Eagle (BME), RPMI1640, Dulbecco's Modified Eagle's Medium: Nutrient Mixture F-10 (DMEM/F-10), Dulbecco's Modified Eagle's Medium: Nutrient Mixture F-12 (DMEM/F-12), α-Minimal Essential Medium (α-MEM), Glasgow's Minimal Essential Medium (G-MEM), Isocove's Modified Dulbecco's Medium (IMDM), and Knockout DMEM, or artificially synthesized media, but the present invention is not limited thereto.

In yet another aspect of the present invention, the present invention provides a method of preventing or treating a diabetic skin disease, which includes administering a pharmaceutical composition including thrombin-treated stem cell-derived exosomes as an active ingredient to an individual.

The term "individual" used herein refers to a subject in need of treatment, and more specifically, a mammal such as a human or a non-human primate, a mouse, a rat, a dog, a cat, a horse, or a cow.

In addition, the present invention provides a use of the pharmaceutical composition for preventing or treating a diabetic skin disease.

Hereinafter, to help in understanding the present invention, exemplary examples will be suggested. However, the following examples are merely provided to more easily understand the present invention, and not to limit the present invention.

### [Examples]

### Example 1. Induction of exosome secretion by thrombin treatment of stem cells

Human cord blood-derived mesenchymal stem cells (3×10⁵) were seeded in a 100-mm culture dish (Orange Scientific cat# 4450200) and cultured for 1 week. After it was confirmed that the cells were saturated and proliferated in the culture plate, the culture medium was replaced with a serum-free culture medium (alpha-MEM media) in which 50 unit/ml thrombin (REYON Pharmaceutical. Co., LTD) was diluted and cultured again for 6 hours. Afterward, the culture solution was divided into centrifuge tubes and centrifuged at 4 °C and 6,000 rpm for 30 minutes, and the supernatant was transferred to a new tube to remove cell debris. Again, the supernatant was ultracentrifuged at 4 °C and 100,000 rpm for 2 to 4 hours, and then the supernatant was further removed to obtain exosomes.

As a result of observing and imaging the exosomes isolated according to the above process under a scanning electron microscope (SEM) and a transmission electron microscope (TEM), as shown in FIG. 1, the exosomes secreted by thrombin stimulation through the above-described method were confirmed.

In addition, to clearly confirm whether the obtained product is an exosome, western blotting was performed to verify the expression of known exosome markers CD9, CD63 and CD81. As a result, as shown in FIG. 2, the product obtained from thrombin-treated stem cells was found to normally express CD9, CD63 and CD81 and thus identified as an exosome.

### Example 2. Verification of therapeutic effect of thrombin-treated stem cell-derived exosome on diabetic dermatopathy

The present inventors conducted the following *in vivo* experiment to verify whether the exosome obtained by the method of Example 1 has a therapeutic effect on a chronic diabetic skin disease.

First, to manufacture a diabetes-induced animal model, after streptozotocin was intraperitoneally administered to an SD rat aged 12 to 16 weeks at 100 mg/kg/body once a day for 4 days, blood was collected from the tail for 2 weeks to measure a blood sugar level. As a result of blood glucose measurement, only rats with a blood glucose level of 300 mg/dL or more were selected and used for the experiment. As shown in FIG. 3A, the normal group used in the experiment was intraperitoneally administered saline under the same conditions, and the blood sugar was measured to be 100 mg/dL, and it was confirmed that the diabetes-induced group had an average blood sugar of 350 mg/dL or more.

Thereafter, diabetic dermatopathy was induced in a diabetes-induced rat by making a wound with a certain size on its back. After making the wound, exosomes (naive exo) isolated from untreated mesenchymal stem cells (naive MSC) and exosomes (thrombin exo) isolated from thrombin-treated mesenchymal stem cells were directly applied (10 µg/each) to each lesion. Here, as a result of measuring the blood glucose for each group of rats corresponding to each experimental group, as shown in FIG. 3B, it was confirmed that there is no difference in blood glucose between groups.

After that, the corresponding site was photographed at intervals of 5 days and the change in the disease area was measured and the treatment effect was compared, as shown in FIG. 4, compared to when each of saline and naive MSC-derived exosomes was treated, when the thrombin-treated MSC-derived exosomes were treated, and the therapeutic effect on the skin lesion was considerably higher. From the above results, it can be seen that exosomes isolated from thrombin-treated MSCs have an excellent therapeutic effect on a diabetic skin disease.

It should be understood by those of ordinary skill in the art that the above descriptions of the present invention are exemplary, and the example embodiments disclosed herein can be easily modified into other specific forms without changing the technical spirit or essential features of the present invention. Therefore, it should be interpreted that the example embodiments described above are exemplary in all aspects, and are not limitative.

### [Industrial Applicability]

Since the exosome-based therapeutic agent according to the present invention is a cell-free formulation and thus does not include DNA, such therapeutic agents have a low risk of cancer and are free of cell surface antigens. Accordingly, such an exosome-based therapeutic agent does not have the issue of transplant rejection and is thus safe. Moreover, as a separation material, rather than cells, the exosome-based therapeutic agent can be developed as an off-the-shelf product and thus can reduce production costs. Accordingly, it is expected that the exosomes derived from thrombin-treated stem cells according to the present invention can be used as a therapeutic agent for various intractable chronic skin diseases that can be caused by diabetes.

## Claims

1. A pharmaceutical composition for preventing or treating a diabetic skin disease, comprising:
thrombin-treated stem cell-derived exosomes as an active ingredient.

2. The composition of claim 1, wherein the stem cells are selected from the group consisting of mesenchymal stem cells, human tissue-derived mesenchymal stromal cells, human tissue-derived mesenchymal stem cells, pluripotent stem cells, and amniotic epithelial cells.

3. The composition of claim 2, wherein the mesenchymal stem cells are derived from the umbilical cord, cord blood, bone marrow, fat, muscle, skin, the amniotic membrane or placenta.

4. The composition of claim 1, wherein the diabetic skin disease is selected from the group consisting of diabetic ulcers, eruptive xanthomatosis, cutaneous infection, diabetic dermopathy, skin fibroma, acanthosis nigricans, pruritus, scleredema diabeticorum, and granuloma annulare.

5. The composition of claim 1, further comprising an auxiliary component selected from the group consisting of a culture medium, a cytokine, a growth factor, and a gene.

6. A pharmaceutical formulation for treating a diabetic skin disease, comprising the composition of claim 1.

7. The formulation of claim 6, which is an injectable type, an infusion type, a spray type, a liquid type, or a patch type.

8. A quasi-drug formulation for improving a diabetic skin disease, comprising thrombin-treated stem cell-derived exosomes as an active ingredient.

9. The formulation of claim 8, which is an external formulation for skin selected from the group consisting of a liquid, an ointment, a cream, a spray, a patch, a gel, and an aerosol.

10. A method of preparing the pharmaceutical composition of claim 1, comprising the following steps:
(a) treating thrombin after stem cell culture;
(b) isolating exosomes from the cell culture of (a); and
(c) preparing a composition containing the exosomes isolated in (b) as an active ingredient.

11. The method of claim 10, wherein the thrombin of (a) is comprised at 1 to 1000 unit/ml in the medium.

12. The method of claim 10, wherein the exosomes of (b) are isolated by centrifugation at 3,000 to 100,000 g for 10 minutes to 5 hours.

13. A method of preventing or treating a diabetic skin disease, comprising:
administering a pharmaceutical composition comprising thrombin-treated stem cell-derived exosomes as an active ingredient to an individual.

14. A use of a pharmaceutical composition, which comprises exosomes derived from thrombin-treated stem cells, for preventing or treating a diabetic skin disease.
